# EUROPEAN PATENT SPECIFICATION

(11) **EP 3 470 068 B1**
(45) Date of publication and mention of the grant of the patent: **25.11.2020**
(21) Application number: 18199646.3
(22) Date of filing: 10.10.2018
(51) Int. Cl.: A61K 38/08, A61K 31/205, A61K 33/28, A61P 31/00, A61K 31/14, A61K 31/305

(54) **ANTIBACTERIAL COMPOSITIONS WITH ENHANCED ACTIVITY**
ANTIBAKTERIELLE ZUSAMMENSETZUNG MIT ERHÖTER AKTIVITÄT
COMPOSITION ANTIBACTÉRIELLE AVEC ACTIVITÉ AMÉLIORÉE

(30) Priority: 11.10.2017 IT 201700114664
(43) Date of publication of application: 17.04.2019
(73) Proprietor: Università degli Studi di Parma, 43121 Parma (IT)
(72) Inventor: CABASSI, Clotilde Silvia, 42123 Reggio Emilia (IT); SALA, Andrea, 41030 Bomporto (Modena) (IT); TADDEI, Simone, 56127 Pisa (IT); FLISI, Sara, 43126 Parma (IT)
(74) Representative: Gerli, Paolo

(56) References cited:
- WO-A1-2016/184855
- WO-A2-2014/102596

### SEQUENCE LISTING

<110> Universita degli Studi di Parma
<120> Antibacterial compositions with enhanced activity
<130> UPR009BEP
<150> IT102017000114664
   <151> 2017-10-11
<160> 9
<170> BiSSAP 1.3.6
<210> 1
   <211> 20
   <212> PRT
   <213> Unknown
<220>
   <223> None
<400> 1
   Lys Leu Ile Pro Ile Leu Ser Lys Thr Ile Pro Ala Ile Lys Asn Leu
   1               5                   10                  15
   Phe Tyr Lys Ile
               20
<210> 2
   <211> 20
   <212> PRT
   <213> Unknown
<220>
   <223> None
<400> 2
   Lys Leu Ile Trp Ile Leu Ser Lys Thr Ile Pro Ala Ile Lys Asn Leu
   1               5                   10                  15
   Phe Tyr Lys Ile
               20
<210> 3
   <211> 20
   <212> PRT
   <213> Unknown
<220>
   <223> None
<400> 3
   Lys Leu Ile Pro Ile Ala Ser Lys Thr Ile Pro Ala Gly Lys Asn Leu
   1               5                   10                  15
   Phe Tyr Lys Ile
               20
<210> 4
   <211> 20
   <212> PRT
   <213> Unknown
<220>
   <223> None
<400> 4
   Lys Leu Ile Pro Ile Ala Ser Lys Thr Ile Pro Ala Gly Lys Asn Leu
   1               5                   10                  15
   Ile Tyr Lys Ile
               20
<210> 5
   <211> 20
   <212> PRT
   <213> Unknown
<220>
   <223> None
<400> 5
   Lys Leu Ile Pro Ile Ala Ser Lys Thr Ile Pro Ala Gly Lys Asn Leu
   1               5                   10                  15
   Leu Tyr Lys Ile
               20
<210> 6
   <211> 20
   <212> PRT
   <213> Unknown
<220>
   <223> None
<400> 6
   Lys Leu Ile Pro Ile Ala Ser Lys Thr Ile Pro Ala Gly Lys Asn Leu
   1               5                   10                  15
   Val Tyr Lys Ile
               20
<210> 7
   <211> 20
   <212> PRT
   <213> Unknown
<220>
   <223> None
<400> 7
   Lys Leu Ile Phe Ile Leu Ser Lys Thr Ile Pro Ala Ile Lys Asn Leu
   1               5                   10                  15
   Phe Tyr Lys Ile
               20
<210> 8
   <211> 20
   <212> PRT
   <213> Unknown
<220>
   <223> None
<400> 8
   Lys Leu Ile Leu Ile Leu Ser Lys Thr Ile Pro Ala Ile Lys Asn Leu
   1               5                   10                  15
   Phe Tyr Lys Ile
               20
<210> 9
   <211> 20
   <212> PRT
   <213> Unknown
<220>
   <223> None
<400> 9
   Lys Leu Ile Tyr Ile Leu Ser Lys Thr Ile Pro Ala Ile Lys Asn Leu
   1               5                   10                  15
   Phe Tyr Lys Ile
               20

## Description

### FIELD OF INVENTION

The present invention relates to the field of antimicrobial products and their use in human and veterinary medicine. New combination products are described having selectively enhanced antibacterial activity, comprising a group of synthetic peptides, selected disinfectants and, optionally, chelating agents.

### STATE OF THE ART

In recent years, the massive use of antimicrobials in human and veterinary medicine has increased the emergence and spread of resistant microorganisms. The situation has worsened due to the lack of investment in the development of new effective antibiotics. It is estimated that every year drug-resistant infections are responsible for the death of at least 25000 patients and cost the EU € 1.5 billion in health costs and loss of productivity. (Official Journal of the European Union, Guidelines for the prudent use of antimicrobials in veterinary medicine - 2015/C 299/04).

The proper use of antimicrobials in human and veterinary medicine is one of the EU's key strategic plans in the fight against antimicrobial resistance.

Therefore, antimicrobial resistance (AMR) is a priority not only for the European Community but also for the Public Health worldwide, where different institutions collaborate through a "One Health" approach, which involves the participation of all sectors and covers all aspects of antimicrobial resistance. The action plan aims first of all to strengthen the prevention and control of AMR in the human, veterinary and food sectors, and to ensure the availability and prolongation of the effectiveness of antimicrobial agents.

For this purpose, it is suggested by European legislation that Universities and other research Institutions prioritize research in the field of antimicrobial resistance, making more efforts for the development of alternative tools for the control of infections, in particular for the development of new classes of antimicrobials.

In all animal species, including pets and livestock, it is recommended to avoid the incorrect use of conventional antibiotics: the use of non-targeted molecules, at sub-therapeutic doses, repeatedly or for inadequate periods of time. Furthermore, the use of antimicrobial agents that tend to favour the propagation of transmissible resistance must be avoided.

The development of antimicrobial molecules, including antimicrobial peptides (AMP) arises from the need to respond to the limitations due to the use of conventional antibiotics. In particular, antimicrobial peptides can be effectively used as primary agents or adjuvants in the treatment of infectious diseases of different districts of human and animal organisms.

Antimicrobial peptides are good alternatives to conventional antibiotics. Examples of such peptides have been described, since 1980, as part of the innate immune system and naturally occurring in all organisms, from bacteria to humans. Currently, research has developed an increasing number of peptides with antimicrobial activity starting from naturally occurring peptide scaffolds. Furthermore, it has been suggested that the development of resistance to AMPs is unlikely, since the mechanism of action on conserved targets as the bacterial membrane is non-specific and a high energy cost would be needed to develop resistance.

A group of synthetic antimicrobial peptides derived from Cardiotoxin 1 of Naja atra is described by the patent application WO 2016/184855 in name of the present Applicant. Antimicrobial peptides belonging to this family, herein named NCP (*Naja atra cardiotoxin peptides*), showed excellent antibacterial activity against Gram positive and Gram negative bacteria, strong antifungal activity against yeasts, antimycobacterial and antiviral activity, together with low cytotoxicity.

The present Applicant has long been involved in the research and development of new antibacterial products with enhanced activity and a diversified range of action, which allow for example a reduction in the relative concentrations of the individual components. The association of antibacterial peptides with other substances that can lead to an enhanced and/or synergistic antimicrobial effect could increase the speed of action, further decrease the cytotoxicity of the individual components and decrease the overall cost of the formulation. On the other hand, antibacterial peptides are labile molecules and their combination with other substances may result in unwanted interactions/reactions, causing e.g. a partial denaturation of the peptide with consequent reduction of antibacterial activity; the addition of other substances may also modify the hydro/lipophilic characteristics of the product and modify the degree of dispersibility and/or the extent of absorption by the bacterium, with consequent unpredictable variation of the antibacterial effect; furthermore, since the antimicrobial peptides are electrically charged, they can easily interact with any associated molecules forming complexes with lesser freedom of movement and/or binding to the target structures on the surface of the bacterium. If successful, these combined formulations would represent a useful example of commercial products for the treatment of animal and human infections.

A characteristic of antibacterial peptides is the broad spectrum of action: it can offer an advantage in the early treatment of infections caused by bacteria not yet identified, or caused by infections due to multiple bacteria; on the other hand, the same characteristic is disadvantageous, leading to an often unnecessary diffusion into the environment of bactericides, not justified by corresponding infections: this favors the development of resistant species. Therefore, the need remains for antibacterial peptides and/or compositions containing them whose antibacterial activities is high but, at the same time, more selected against sub-categories of bacteria: therapeutic treatments based on such products or compositions would be advantageous for patients infected with bacteria belonging to said sub-categories, avoiding at the same time a useless environmental dispersion of bactericides, not useful for the patient and that favors the development of resistant bacterial species.

### SUMMARY

It has now been discovered that a homogeneous and limited group of antimicrobial peptides derived from Naja atra cardiotoxins, when combined with selected disinfectants and, optionally, chelating agents, has a selectively high and generally synergistic effect against Gram negative bacteria, thus resulting of high interest in the prevention and/or treatment of infections caused by Gram negative bacteria.

The group of peptides of the invention, linear and containing 20 amino acids, is defined by the following structure (I):

KLI-X1-SKTIPA-X2-KNL-X3-YKI (I)

In this structure the variable residues X1, X2 and X3 have the following meanings:
- X1 is a linear sequence of 3 hydrophobic amino acids
- X2 is a single amino acid selected from isoleucine (I) and glycine (G);
- X3 is a single hydrophobic amino acid and are further defined by the claims.

The disinfectants that may be associated with the above mentioned peptides of formula (I) belong specifically to the class of quaternary ammonium compounds (QACs) and/or to the class of organomercuric compounds and are selected from benzalkonium chloride and thimerosal. These selected classes of compounds were found to be effective in providing a combined product with selectively enhanced and generally synergistic effect against Gram negative bacteria. These effects are further strengthened in the presence of a chelating agent.

The invention therefore relates to a new combination of: (a) one or more peptides of formula (I) as defined above, (b) one or more quaternary ammonium and/or organomercuric disinfectant selected from benzalkonium chloride and thimerosal (c) optionally, one or more chelating agents. The invention also concern the above mentioned combination for use in therapy, with particular effectiveness in the prevention and/or treatment of infections caused by Gram-negative bacteria.

The invention also extends to pharmaceutical compositions for medical or veterinary use comprising: (a) one or more peptides of formula (I) as defined above, (b) one or more quaternary ammonium and/or organomercuric disinfectants as defined above; (c) optionally, a chelating agent; such compositions may further include one or more co-formulants conventionally used in the field, selected according to the type of application desired. The invention also relates to the above mentioned compositions in the prevention and/or treatment of diseases caused by Gram-negative bacteria.

### DESCRIPTION OF FIGURES

FIGURE 1: percent inhibition of the growth of Gram negative and Gram positive reference bacterial strains caused by the NCP-3 peptide, Benzalkonium chloride, Tris/EDTA and their combinations, as a function of time (expressed in minutes, from 0 to 120).
FIGURE 2: percent inhibition of the growth of Gram negative and Gram positive reference bacterial strains caused by the NCP-3 peptide, Thimerosal, Tris/EDTA and their combinations, as a function of time (expressed in minutes, from 0 to 120).
FIGURE 3: percent inhibition of the growth of reference fungal strains caused by the NCP-3 peptide, Benzalkonium chloride, Tris/EDTA and their combinations, as a function of time (expressed in minutes, from 0 to 120).
FIGURE 4: percent inhibition of the growth of reference fungal strains caused by the NCP-3 peptide, Thimerosal, Tris/EDTA and their combinations, as a function of time (expressed in minutes, from 0 to 120).

### DETAILED DESCRIPTION

The term "combination" used herein generally refers to products and their applications involving the combination of the components of the combination itself: this combination is obtainable either by physical combination its components to form a single product containing them (e.g. a tablet containing them, homogeneously mixed together or contained in different areas of the tablet); or it can be achieved by formulating the components of the combination in separate products but expressly suited for combined use; this can be achieved, for example, by supplying a kit of parts or a combined administration procedure which provide for the combined use of the above mentioned components.

The terms "disinfectant" or "disinfectant compound" or "disinfectant agent", herein understood as synonyms, refer to a compound having antibacterial activity, that is able to partially or totally inhibit the growth of one or more bacteria.

The terms "chelating" or "chelating compound" or "chelating agent", here understood as synonyms, refer to a compound capable of binding or sequester one or more ions, preferably metallic, by means of suitable bonds, e.g. coordination bonds; such compounds may be molecular complexes which include the above mentioned ions, or may form such complexes in the presence of the above mentioned ions.

The term "treatment" refers to the ability to obtain a therapeutic benefit to a patient suffering from an infectious diseases, achieving an improvement in the patient's condition (reduction or elimination of symptoms, slowing or stopping the course of the disease).

The term "prevention" refers to the ability to obtain a therapeutic benefit on a healthy patient not yet suffering from a disease, preventing a pathological evolution towards this disease (slowing or blocking of the onset of the disease), these effects being found at least during the administration of the treatment and possibly thereafter.

The term "infection" or its synonym "infectious disease" means the invasion, colonization and/or the multiplication of a microorganism in or on another host organism.

"Subject" or "patient" is any multicellular organism, including a human, an animal, a plant or an insect that can be infected with a microorganism. In the case of administrations for the purpose of treatment of a disease, the patient is infected at the time of administration; in the case of administrations for the purpose of prevention of a disease, the patient is not infected at the time of administration. Preferably, the subject is a human or animal organism.

"Peptide" is defined by a multiplicity of amino acid residues joined by peptide bonds; it is the same as "polypeptide" and "protein" and can be used interchangeably.

Amino acids are reported herein using the standard abbreviations commonly used for sequence description (single letter codes). All the amino acids referred to in the present text can be either in the D- or L-form; preferably, they are predominantly (i.e. above 50%) or totally in the L- form. A preferred aspect relates to peptides consisting of at least 80%, for example 90%, or more preferably 91-100%, for example 100%, of L-amino acids. Another preferred aspect relates to peptides constituted of at least 80%, for example 90%, or more preferably 91-100%, for example 100%, of D-amino acids.

In this document, the term "excipient" refers to a compound or mixture thereof that is optimal for use in a formulation prepared for the treatment of a specific infectious pathology or conditions associated therewith. For example, an excipient for use in a pharmaceutical formulation should not generally cause an adverse response in a subject. The excipient, as previously described, must not significantly inhibit the relevant biological activity of the active compound. For example, an excipient does not inhibit the antimicrobial activity of an antimicrobial peptide of the present invention or a variant thereof. An excipient, for example saline phosphate buffer, can simply provide a buffering activity to keep the active compound at a suitable pH to exert its biological activity. Alternatively or in addition, the excipient may comprise a compound, e.g. a protease inhibitor, which increases peptide activity or half-life. In another example, the excipient may include or be itself an additional antimicrobial molecule and/or an anti-inflammatory molecule.

The peptides of the invention are linear, have a lenght of 20 amino acids and have the following structure (I).

KLI-X1-SKTIPA-X2-KNL-X3-YKI (I)

wherein X1, X2 and X3 are defined according to the claims.

Also disclosed are peptides of formula (I) wherein
- X1 is a linear sequence of 3 hydrophobic amino acids
- X2 is a single amino acid selected from isoleucine (I) and glycine (G);
- X3 is a single hydrophobic amino acid
In the said formula (I), the initial first K amino acid represents the - HN₂ terminal region, while the final I amino acid represents the -COOH terminal region.

The hydrophobic amino acids usable for position X1 and X3 may be selected among those commonly known as "hydrophobic", that is specifically: alanine (A), phenylalanine (F), leucine (L), isoleucine (I), proline (P), tyrosine (Y), tryptophan (W) e valine (V).

The hydrophobic amino acids which compose X1, selected among those indicated above, may be to each other: equal, partly different, or all different; preferred examples for X1 are the following tripeptides: PIL, WIL, PIA, FIL, LIL e YIL.

The single amino acid X3 may be selected among those hydrophobic amino acids listed above, independently of the amino acids present in X1; preferred examples of amino acids for X3 are: F, I, L, V.

The structural formula (I) as defined above is inherently homogeneous, with structural peculiar characteristics. Therein are distinguished, on a 20-amino acids specific length, three hydrophobic regions (residues LIX1, IPA, LX3Y): they contain exclusively hydrophobic amino acids, thus forming extended hydrophobic areas highly homogeneous from this point of view, of which the first area (LIX1) is constituted by 5 amino acids, thus being particularly broad. The above-mentioned hydrophobic zones are alternated with charged areas (residues SKT and IKN) containing basic amino acids and hydrogen bonds-forming amino acids. The resulting peptide shows a high tendency to take a spatial α helix conformation. The peptides of (I) formula are easy to be synthetized, highly effective, proteolytically stable, substantially salt resistant, non-hemolytic and with low toxicity for eukaryotic cells.

Subject to their composition with sole hydrophobic amino acids, there are no further limitations in the selection of the specific amino acids present in X1 and X3; likewise, the specific amino acids present in X2 may be freely chosen among I and G amino acids. Nevertheless, the present invention comprises some preferred embodiments.

In a preferred embodiment, in the above-mentioned formula (I): X1 is selected from PIL or WIL, X2 is as indicated above and X3 is F.

In a second preferred embodiment: X1 is PIA, X2 is as indicated above and X3 is selected from F, I, L, V.

In a third preferred embodiment: in the above-mentioned formula (I), X1 is selected from FIL, LIL or YIL, X2 is as above indicated and X3 is F.

Particularly preferred examples of peptides according to the above-mentioned formula (I) are selected from the following:
(i) KLIPILSKTIPAIKNLFYKI, herein also referred as NCP2;
(ii) KLIWILSKTIPAIKNLFYKI, herein also referred NCP3;
(iii) KLIPIASKTIPAGKNLFYKI, herein also referred NCP1A;
(iv) KLIPIASKTIPAGKNLIYKI, herein also referred NCP1B;
(v) KLIPIASKTIPAGKNLLYKI, herein also referred NCP1C;
(vi) KLIPIASKTIPAGKNLVYKI, herein also referred NCP1D;
(vii) KLIFILSKTIPAIKNLFYKI, herein also referred NCP3A;
(viii) KLILILSKTIPAIKNLFYKI, herein also referred NCP3B;
(ix) KLIYILSKTIPAIKNLFYKI, herein also referred NCP3C;
the above-indicated sequences (i) - (ix) and their *sequence listings* (respectively, SEQ.ID.NOs from 01 to 09) are described in the co-pending application WO 2016/184855 in name of the present Applicant.

The disinfectant belonging to the quaternary ammonium compounds, usable in combination with the peptides of formula (I), are cationic compounds with surfactant properties, widely known on the market for over 100 years, known also as *QACs- quaternary ammonium compounds*; commonly considered to be not very effective against Gram-negative bacteria, they unexpectedly generated a significant and selective increase in activity towards such bacteria when combined with the peptides of the above-defined formula (I).

Said quaternary ammonium disinfectants typically comprise a quaternary cationic nitrogen atom substituted by alkyl groups, (e.g., C8-C18, optionally interrupted by one or more -O-) and/or aryl or arylalkyl groups (e.g., phenyl or benzyl), and their derivatives; alternatively, said nitrogen atom is a quaternarized heterocyclic nitrogen (e.g. belonging to a pyridine ring).

As positive cations, said QACs are associated with a suitable negative counterion (preferably chloride or bromide).

Organomercuric disinfectants, i.e. organic compounds comprising one or more mercury atoms, are also commonly known in the art. The best-known example, is Thimerosal (also known as Thiomersal or sodium-ethylmercury-thiosalicylate) The disinfectant according to the invention is selected from benzalkonium chloride and thimerosal.

In a preferred variant, chelating agents may be present in combination with the above-mentioned ingredients.

In fact, it has been found that the increased activity against Gram-negative bacteria exerted by the association of the peptides of formula (I) with said quaternary and/or organomercuric ammonium disinfectants is further increased in the presence of one or more chelating agents. Chelating agents are commonly known in the art. Examples of such compounds are ethylenediamine tetraacetic acid and its salts (EDTA, EDTA/Tris), citric acid and its salts, etc.

The selection of the disinfectant and any chelating agent in the above-indicated classes is also made taking into account their compatibility with the biological treatment target (human, animal, etc.).

As shown in the experimental section, the combination of the invention is selectively and highly effective in inhibiting the growth of Gram-negative bacteria, also showing clear evidences of synergy; as a consequence, the combination may be conveniently used in human or veterinary medicine in the treatment and/or in the prevention of diseases caused by said Gram negative bacteria; on the other hand, the same combination was much less effective against Gram positive bacteria and/or fungi, thus confirming the selectivity found.

A preferred, though examples of Gram negative bacteria being target of the present treatment are those belonging to one or more of the genera:
*Acinetobacter, Burkholderia, Cardiobacterium, Enterobacter, Escherichia, Flavobacterium, Haemophilus, Helicobacter, Klebsiella, Legionella, Moraxella, Mycoplasma, Neisseria, Proteus, Pseudomonas, Pseudoxanthomonas, Rickettsia, Salmonella, Shigella, Vibrio,* ecc. Preferred target of the treatment are the genera *Escherichia, Moraxella, Acinetobacter, Enterobacter, Klebsiella, Burkholderia, Pseudomonas;* in particular, the bacterial species *Pseudomonas aeruginosa, Escherichia coli, Moraxella catarrhalis, Acinetobacter* baumanii, *Enterobacter cloacae, Klebsiella pneumoniae* subsp. *pneumoniae* e *Burkholderia cepacia.* Specifically preferred target are the genera *Escherichia* e *Pseudomonas,* in particular the bacterial species *Escherichia coli* e *Pseudomonoas aeruginosa.*

The present treatment (or prevention) is here meant extended to any pathological condition in which an anomalous presence of Gram negative bacteria is found (or is to be expected) in humans or animals. The present treatment may be employed in the treatment of infections in various district of the human or animal body (for example, pulmonar, gastrointestinal, ocular, urinary), skin infection and medical or surgical diseases, possibly complicated by further superinfections.

Therefore, the present invention concerns, as such, the combination of one or more peptides having the above defined formula (I) with one or more disinfectant selected from benzalkonium chloride and thimerosal and, optionally, one or more chelating agents.

The invention also concerns pharmaceutical compositions comprising the combination of one or more peptides having the above defined formula (I), with one or more disinfectant selected from benzalkonium chloride and thimerosal and, optionally, one or more chelating agents.

The invention also concerns the combination of one or more peptides having the above defined formula (I), with one or more disinfectant selected from benzalkonium chloride and thimerosal and, optionally, one or more chelating agents, for general use in therapy.

The invention also concerns the combination of one or more peptides having the above defined formula (I), with one or more disinfectant selected from benzalkonium chloride and thimerosal and, optionally, one or more chelating agents, for use in the treatment of diseases caused by Gram negative bacteria.

The invention also concerns the use of the combination of one or more peptides having the above defined formula (I), with one or more disinfectant selected from benzalkonium chloride and thimerosal and, optionally, one or more chelating agents, in the preparation of a drug for the treatment and/or prevention of diseases caused by Gram-negative bacteria.

Mammals, birds, reptiles, amphibia and, in general, other animals may be treated with the peptides described in the present invention. Mammals and birds include, humans, dogs, cats, pet birds and farm animals, such as horses, cattle, sheep, goats, pigs, chickens and turkey and poultry.

The above indicated pharmaceutical compositions containing the combination of the present invention may contain appropriately selected excipients, according to known teachings, depending on the chosen form of administration, the site of administration and the desired characteristics of absorption and distribution of the product. Said pharmaceutical compositions can be prepared by procedures described in the art and using known and readily available excipients.

The combinations of the invention may be formulated in the form suitable for any route of administration, e.g. oral, topical, endomammary, parenteral, intramuscular, subcutaneous, intraperitoneal or intravenous, etc. They can take the form of an aqueous solution, an anhydrous form or a dispersion, or alternatively an emulsion, a gel, a suspension, an ointment, a cream or an ointment. They may be included in suspension, solutions or emulsions in oily vehicles or in water and may contain useful agents for suspending, stabilizing and/or agents favouring the dispersion. They can also be formulated in solid form e.g. such as tablets, capsules, microcapsules, pellets, granules, microgranules, powder, etc.

In the case of treatments of humans or animals, the combination of the present invention is conveniently produced and administered in the form of one or more dosage units. Said dosage units may comprise said peptide of formula (I) in amounts generally included e.g. between 0.2 µg and 5 mg.

Said dosage units may further comprise said quaternary ammonium disinfectant in amounts generally comprised e.g. between 0.003 mg and 20 mg and/or organomercuric compounds in quantities generally comprised e.g. between 0.03 mg and 400 mg. Said dosage units can further optionally comprise said chelating agent in amounts generally comprised e.g. between 0.5 mg and 400 mg. If more than one peptide, or more than one disinfectant, or more than one chelating agent are used, the above weight ranges refer to the sum of the weights of the respective compounds belonging to each of the three categories.

The above-mentioned peptides having the above defined formula (I), disinfectants and any chelating agents can be co-formulated in the same dosage unit: in this case they can be present as a mixture or separated from one another in different areas of the same dosage unit (e.g. core and coating of a tablet). Alternatively, the above components may be contained in independent dosage units, but presented to the patient for a combined administration; in this case they can be supplied e.g. in the form of a kit of parts formed by separate units containing respectively the above mentioned peptides, disinfectants and any chelating agents; such kits preferably include appropriate instructions for the combined administration of the relative components.

The combination of the present invention can be effectively used as primary agents or adjuvants in the treatment and/or the prevention of infectious diseases of various districts of the human or animal organism.

Moreover, the combinations of the present invention may be employed in the treatment of infections of plant organism. The combinations of the present invention may also be useful in the treatment of associated infections, generally of the skin, e.g. ulcers and lesions, skin wounds, cuts or burns.

A further preferred aspect of the invention indicates that the above indicated combination are useful in the treatment of bacterial skin infection of pyoderma in different districts including, but not limited to, mammary gland, ear, eye, foot, hoof. Another aspect involves the use, collectively or individually, of the peptides of the invention in the treatment of (clinical or surgical) diseases complicated by further bacterial superinfections including, without limitation, the associated: mucosal infections, gastrointestinal tract infections, urogenital infections, infections of the urinary tract (for example, pyelonephritis, cystitis, urethritis) or respiratory infections.

The present invention is further described by the following, examples.

### EXPERIMENTAL SECTION

### Example 1: determination of the ability to inhibit growth in liquid culture by the NCP-3 peptide (aminoacidic sequence KLIWILSKTIPAIKNLFYKI), the benzalkonium chloride and thimerosal disinfectants and chelating agents (Tris / EDTA) on reference bacterial and fungal strains.

This example evaluates the antibacterial spectrum of activity of the tested molecules against representative Gram-negative bacteria (*Escherichia coli* ATCC 25922, *Pseudomonas aeruginosa* ATCC 27853); Gram-positive (*Staphylococcus aureus* ATCC 25923, Methicillin Resistant *Staphylococcus aureus* (MRSA) ATCC 43300); fungi (*Candida albicans* ATCC 11006 or *Malassezia pachydermatis* DSMZ 6172). For each strain the Minimal Inhibitory Concentration (MIC) was determined.

### METHODS

### a) Bacterial inoculum preparation

For each bacterial strain 3-5 morphologically similar colonies from fresh cultures were taken, inoculated in *Brain Heart Infusion* broth medium and then incubated at 37°C in agitation at 225 r.p.m. for about 3-4 hours. Subsequently, the bacterial suspension was centrifuged at 1000 r.p.m. for 20 minutes and the obtained pellet was resuspended in phosphate buffer 10 mM. Turbidity of the bacterial suspension was measured by evaluating the absorbance using a spectrophotometer at 600 nm. The range 0,08-0,13 equates to a concentration of about 10⁸ CFU/ml. The bacterial suspension was diluted 1:100 in phosphate buffer 10 mM, then, within 30 minutes, 50 µl of this bacterial suspension (10⁶ CFU/ml) were inoculated in each well of the microtiter plate to obtain a final bacterial concentration of about 5 x 10⁵ CFU/ml.

### b) Fungal inoculum preparation

For each fungal strain 3-5 morphologically similar colonies were collected from fresh cultures, inoculated in *Brain Heart Infusion* broth medium and then incubated at 37°C in agitation at 225 r.p.m. for 6-8 hours. Subsequently, the fungal suspension was centrifuged at 1000 r.p.m. for 20 minutes and the obtained pellet was resuspended in phosphate buffer 10 mM and diluted to a turbidity equal to 5 of McFarland standard, corresponding to a concentration of 10⁸ CFU/ml. Fungal suspension was diluted 1:100 in phosphate buffer 10 mM, then, within 30 minutes, 50 µl of this bacterial suspension (10⁶ CFU/ml) were inoculated in each well of microtiter plate to obtain a final concentration of about 5 x 10⁵ CFU/ml.

### c) Serial microdilution

The different molecules were tested for their ability to inhibit bacterial and fungal growth in broth culture. Single molecules were diluted in phosphate buffer 10 mM with 40% of Müller Hinton broth. 50 µl of diluted molecules were added to microtiter U plate wells with phosphate buffer and 40% of Müeller Hinton broth and a suspension of one of these strains: *Escherichia coli* ATCC 25922, *Pseudomonas aeruginosa* ATCC 27853, *Staphylococcus aureus* ATCC 25923, MRSA ATCC 43300, *Candida albicans* ATCC 11006 or *Malassezia pachydermatis* DSMZ 6172.

For bacterial strains the different molecules were tested at the following concentrations:
- NCP-3: from 0,2 µg/ml to 50 µg/ml
- Benzalkonium chloride: from 0,00008% to 0,04%
- Thimerosal: from 0,0008% to 0,4%
- Tris/EDTA: from 0,4%/0,1% to 0,0008%/0,0002%

For fungal strains the different molecules were tested at the following concentrations:
- NCP-3: from 0,2 µg/ml to 50 µg/ml
- Benzalkonium chloride: from 0,005% to 0,00001%
- Thimerosal: from 0,2% to 0,0004%
- Tris/EDTA: from 25,6/6,4% to 0,05/0,0125%

Bacterial and fungal growth controls were cultured in absence of antimicrobial molecules. Microtiter plates were incubated 24 or 48 hours (bacterial and fungal strains, respectively) at 37°C and subsequently MIC was determined for each molecule against each strain.

### RESULTS

Table 1 reports MIC values for different bacterial and fungal strains and the different molecules tested. Peptide of NCP-3 formula (I), if used alone, results more effective against Gram positive bacteria, with MIC values of 3,2 µg/ml, compared to Gram negative, of which MIC value was 6,3 µg/ml. Disinfectants, used alone, shows good efficacy towards to each bacterial strain tested and, in particular, MIC value is between 0,00031% and 0,00125% for Benzalkonium chloride and between 0,0031% and 0,0125% for Thimerosal. Tris/EDTA, used alone, shows inhibition on each bacterial strain at concentration between 0,00125/0,0031% and 0,1/0,025%.

**TABLE 1: MIC values for different molecules towards reference bacterial strains, obtained through serial microdilutions**

| Bacterial strain | NCP-3 MIC | Benzalkonium chloride MIC | Thimerosal MIC | Tris/EDTA MIC |
|---|---|---|---|---|
| *E. coli* ATCC 25922 | 6,3 µg/ml | 0,00063% | 0,0031 % | 0,0125/0,0031% |
| *P. aeruginosa* ATCC 27853 | 6,3 µg/ml | 0,00031 % | 0,1% | 0,1/0,025% |
| *S. aureus* ATCC 25923 | 3,1 µg/ml | 0,00016% | 0,025% | 0,0125/0,0031% |
| MRSA ATCC 43300 | 6,3 µg/ml | 0,00031 % | 0,0031 % | 0,0125/0,0031% |
| *C. albicans* ATCC 11006 | 12,5 µg/ml | 0,00125% | 0,0063% | 1,6/0,4% |
| *M*. *pachydermatis* DSMZ 6172 | 3,1 µg/ml | 0,00031% | 0,0016% | 1,6/0,4% |

### Example 2: determination of the spectrum of activity and efficacy of NCP-3 peptide (aminoacidic sequence KLIWILSKTIPAIKNLFYKI), of benzalkonium chloride and thimerosal disinfectants and chelating agents (Tris / EDTA) on reference bacterial and fungal strains.

This example evaluates the antibacterial spectrum of activity of tested molecules against *Escherichia coliATCC* 25922, *Pseudomonas aeruginosa* ATCC 27853, *Staphylococcus aureus* ATCC 25923, Methicillin Resistant *Staphylococcus aureus* (MRSA) ATCC 43300, *Candida albicans* ATCC 11006 or *Malassezia pachydermatis* DSMZ 6172. For each strain and molecules, LD90 and where possible LD99 were determined.

### METHODS

### a) Bacterial inoculum preparation

For each bacterial strain 3-5 morphologically similar colonies were collected from fresh cultures, inoculated in *Brain Heart Infusion* broth medium and then incubated at 37°C in agitation at 225 r.p.m. for about 3-4 hours. Subsequently, the bacterial suspension was centrifuged at 1000 r.p.m. for 20 minutes and the obtained pellet was resuspended in phosphate buffer 10 mM. Turbidity of the bacterial suspension was measured by evaluating the absorbance using a spectrophotometer at 600 nm. The range 0,08-0,13 equates to a concentration of about 10⁸ CFU/ml. the bacterial suspension was diluted 1:100 in phosphate buffer 10 mM, then, within 30 minutes, 50 µl of this bacterial suspension (10⁶ CFU/ml) were inoculated in each well of the microtiter plate to obtain a final bacterial concentration of about 5 x 10⁵ CFU/ml.

### b) Fungal inoculum preparation

For each fungal strain 3-5 morphologically similar colonies were collected from fresh cultures, inoculated in *Brain Heart Infusion* broth medium and then incubated at 37°C in agitation at 225 r.p.m. for 6-8 hours. Subsequently, the fungal suspension was centrifuged at 1000 r.p.m. for 20 minutes and the obtained pellet was resuspended in phosphate buffer 10 mM and diluted to a turbidity equal to 5 of McFarland standard, corresponding to a concentration of 10⁸ CFU/ml. The fungal suspension was diluted 1:100 in phosphate buffer 10 mM, then, within 30 minutes, 50 µl of this bacterial suspension (10⁶ CFU/ml) were inoculated in each well of microtiter plate to obtain a final concentration of about 5 x 10⁵ CFU/ml.

### c) Serial microdilution

Different molecules were tested for their ability to inhibit bacterial and fungal growth in broth culture. 50 µl of molecules at the indicated below concentrations were added to microtiter U plate 96 wells with phosphate buffer and a suspension of one of these strains: *Escherichia coli* ATCC 25922, *Pseudomonas aeruginosa* ATCC 27853, *Staphylococcus aureus* ATCC 25923, MRSA ATCC 43300, *Candida albicans* ATCC 11006 or *Malassezia pachydermatis* DSMZ 6172.

The following concentrations of the different molecules were tested:
- NCP-3: from 0,2 µg/ml to 50 µg/ml
- Benzalkonium chloride: from 0,00008% to 0,04%
- Thimerosal: from 0,0008% to 0,4%
- Tris/EDTA: from 0,4%/0,1% to 0,0008%/0,0002%

Bacterial and fungal growth controls were cultured in absence of antimicrobial molecules.

### d) Agar plating

The bacterial or fungal suspension has been contacted for two hours with the different antimicrobial molecules at 37°C. After incubation, 20 µl of solution were withdrawn from each dilution and plated on agar medium, Columbia with 5% of bovine erythrocytes for bacterial strains and Sabouraud agar for fungal strains. The *inoculum* was uniformly distributed on agar plates with a sterile disposable handle. After incubation at 37°C for 24 hours for bacterial strains and 48 hours for fungal strains, colonies (CFU) were counted.

### RESULTS

Table 2 reports the values of LD90 and LD99 for different strains and molecules. NCP-3, used alone, results strongly effective against every strain tested, in particular against *M. pachydermatis* (LD99 equal to 3,2 µg/ml). Among the tested disinfectants, benzalkonium chloride, used alone, results the one with lowest LD99 towards bacterial strains (between 0,00063% and 0,00031%); with respect to yeasts, the LD99 of Thimerosal is lower than the one of benzalkonium chloride (LD99 between 0,0063% and 0,0008%). For what concern Tris/EDTA, the bactericidal effect (LD99) is obtained with concentrations between 0,0125/0,0031% and 0,2/0,05%. With respect to yeasts it was not possible to show a remarkable antimicrobial effect and LD90 results >0,4/0,1% towards either *C. albicans* or *M. pachydermatis.*

**TABLE 2: LD90 and LD99 values towards bacterial and fungal reference strains, obtained with microdilution broth tests and subsequently plated on agar solid medium.**

| | NCP-3 (µg/ml) LD90 | NCP-3 (µg/ml) LD99 | Benzalkonium chloride (%) LD90 | Benzalkonium chloride (%) LD99 |
|---|---|---|---|---|
| *E. coli* ATCC 25922 | 6,3 | 12,5 | >0,00031 | 0,00063 |
| *P. aeruginosa* ATCC 27853 | 6,3 | 12,5 | 0,00016 | 0,00031 |
| *S. aureus* ATCC 25923 | >1,6 | 6,3 | >0,00016 | 0,00031 |
| MRSA ATCC 43300 | 6,3 | 12,5 | 0,00031 | 0,00063 |
| *C. albicans* ATCC 11006 | >6,3 | 12,5 | >0,00063 | <0,0125 |
| *M. pachydermatis*s DSMZ 6172 | >1,6 | 3,2 | 0,00016 | <0,00031 |

| | Thymerosal (%) LD90 | Thymerosal (%) LD99 | Tris/EDTA (%) LD90 | Tris/EDTA (%) LD99 |
|---|---|---|---|---|
| *E. coli* ATCC 25922 | >0,0016 | 0,0031 | 0,025/0,0063 | 0,05/0,0125 |
| *P. aeruginosa* ATCC 27853 | >0,05 | 0,1 | 0,1/0,025 | 0,2/0,05 |
| *S. aureus* ATCC 25923 | >0,0125 | 0,025 | 0,0063/0,0016 | 0,0125/0,0031 |
| MRSA ATCC 43300 | >0,0016 | 0,0031 | 0,0063/0,0016 | 0,0125/0,0031 |
| *C. albicans* ATCC 11006 | >0,0031 | 0,0063 | 1,6/0,4 | ND |
| *M*. *pachydermatis* DSMZ 6172 | >0,0004 | 0,0008 | 1,6/0,4 | ND |

### Example 3: evaluation of bactericidal activity of different antimicrobial molecules, individuality or in combination (Time killing assays).

This example evaluates the antimicrobial activity of the different molecules tested, individually or in combination, in function of time. In particular, NCP-3, Benzalkonium chloride, Thimerosal and Tris/EDTA were tested throughout a two-hours contact period with *Escherichia coli* ATCC 25922, *Pseudomonas aeruginosa* ATCC 27853, *Staphylococcus aureus* ATCC 25923, Methicillin Resistant *Staphylococcus aureus* (MRSA) ATCC 43300, *Candida albicans* ATCC 11006 or *Malassezia pachydermatis* DSMZ 6172. Associations were tested with NCP-3, Tris/EDTA and one of the concerned disinfectants, with concentrations around Minimal Bactericidal Concentration (MBC) for each substance.

### METHODS

### a) Bacterial inoculum preparation

For each bacterial strain 3-5 morphologically similar colonies from fresh cultures were taken, inoculated in *Brain Heart Infusion* broth medium and then incubated at 37°C in agitation at 225 r.p.m. for about 3-4 hours. Subsequently, the bacterial suspension was centrifuged at 1000 r.p.m. for 20 minutes and the obtained pellet was resuspended in phosphate buffer 10 mM. Turbidity of the bacterial suspension was measured by evaluating the absorbance using a spectrophotometer at 600 nm. The range 0,08-0,13 equates to a concentration of about 10⁸ CFU/ml. the bacterial suspension was diluted 1:100 in phosphate buffer 10 mM, then, within 30 minutes, 100 µl of this bacterial suspension (10⁶ CFU/ml) were inoculated in each well of the microtiter plate to obtain a final bacterial concentration of about 5 x 10⁵ CFU/ml.

### b) Fungal inoculum preparation

For each fungal strain 3-5 morphologically similar colonies were collected from fresh cultures, inoculated in *Brain Heart Infusion* broth medium and then incubated at 37°C in agitation at 225 r.p.m. for 6-8 hours. Subsequently, the fungal suspension was centrifuged at 1000 r.p.m. for 20 minutes and the obtained pellet was resuspended in phosphate buffer 10 mM and diluted to a turbidity equal to 5 of McFarland standard, corresponding to a concentration of 10⁸ CFU/ml. The fungal suspension was diluted 1:100 in phosphate buffer 10 mM, then, within 30 minutes, 100 µl of this bacterial suspension (10⁶ CFU/ml) were inoculated in each well of microtiter plate to obtain a final concentration of about 5 x 10⁵ CFU/ml.

### c) Time killing assay

Molecules or their mixtures were tested at the following concentrations (diluted in phosphate buffer 10 mM):
- NCP-3: 6,3 µg/ml
- Benzalkonium chloride: 0,00063%
- Thimerosal: 0,0063%
- Tris/EDTA: 0,8/0,2%
- NCP-3 + Benzalkonium chloride: 0,4 µg/ml + 0,00063%
- NCP-3 + Thimerosal: 0,4 µg/ml + 0,0063%
- NCP-3 + Tris/EDTA: 0,4 µg/ml + 0,4/0,1%
- Tris/EDTA + Benzalkonium chloride: 0,4/0,1% + 0,00063%
- Tris/EDTA + Thimerosal: 0,4/0,1% + 0,0063%
- NCP-3 + Tris/EDTA + Benzalkonium chloride: 0,4 µg/ml + 0,4/0,1% + 0,00063%
- NCP-3 + Tris/EDTA + Thimerosal: 0,4 µg/ml + 0,4/0,1% + 0,0063%

For each listed combination was set a well of a microtiter plate with 100 µl of microbial suspension and 100 µl of molecule/s to test, properly diluted. Growth controls were set up in absence of antimicrobial molecules. The plate was incubated at 37°C for all the time of experiment.

After 1-3-5-10-15-60-90-120 minutes of contact between bacterial/fungal cells and molecules, 20 µl of solution from each well were plated on Columbia agar plates with 5% of bovine erythrocytes for bacterial strains and Sabouraud agar for fungal strains. The *inoculum* was uniformly distributed on agar plates with a sterile disposable handle. After incubation at 37°C for 24 hours for bacterial strains and 48 hours for fungal strains, colonies (CFU) were counted.

### RESULTS

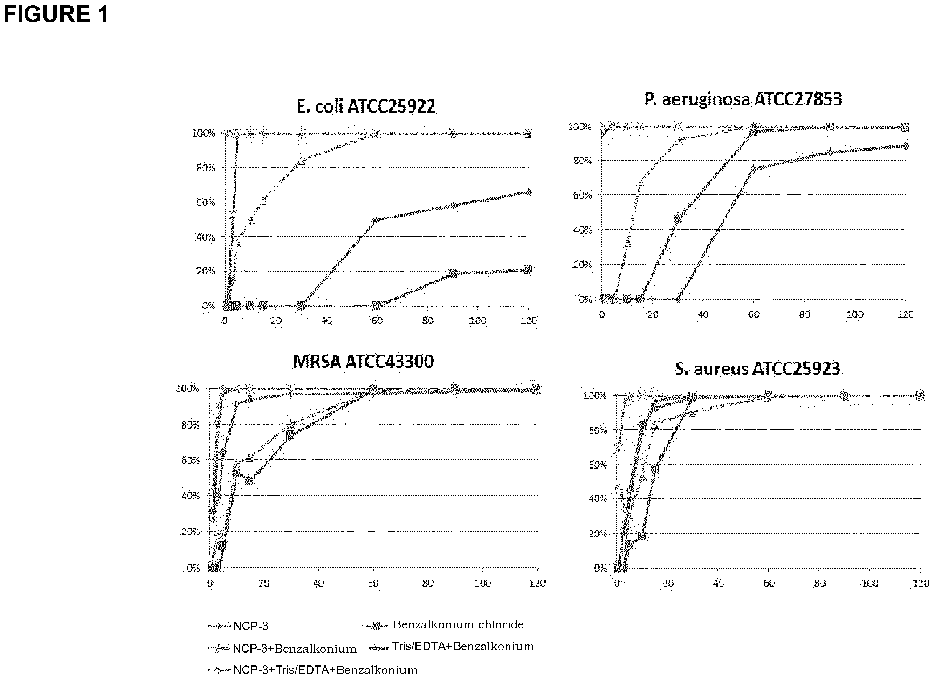

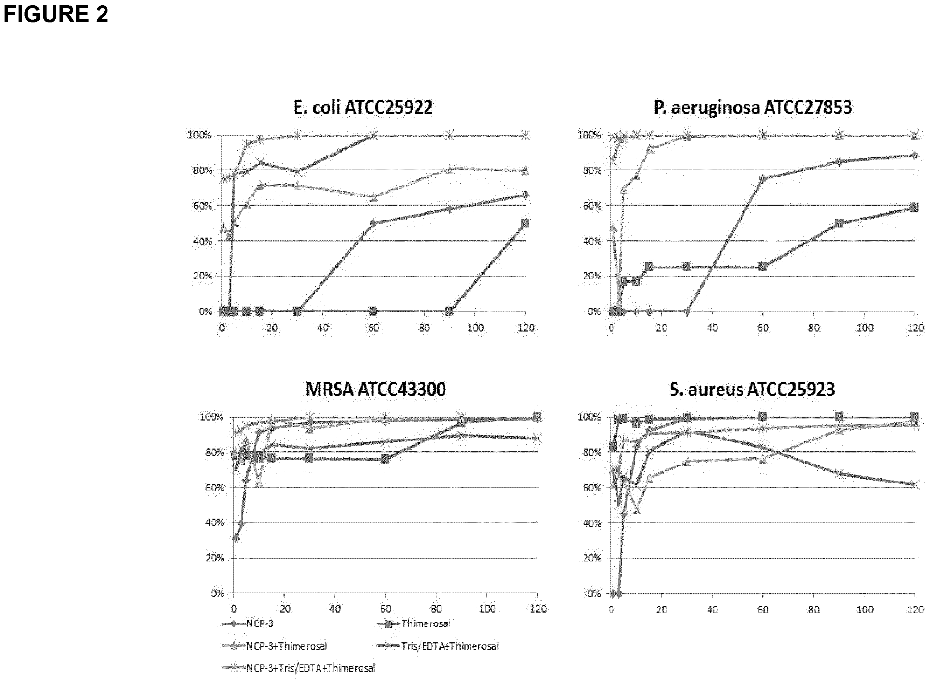

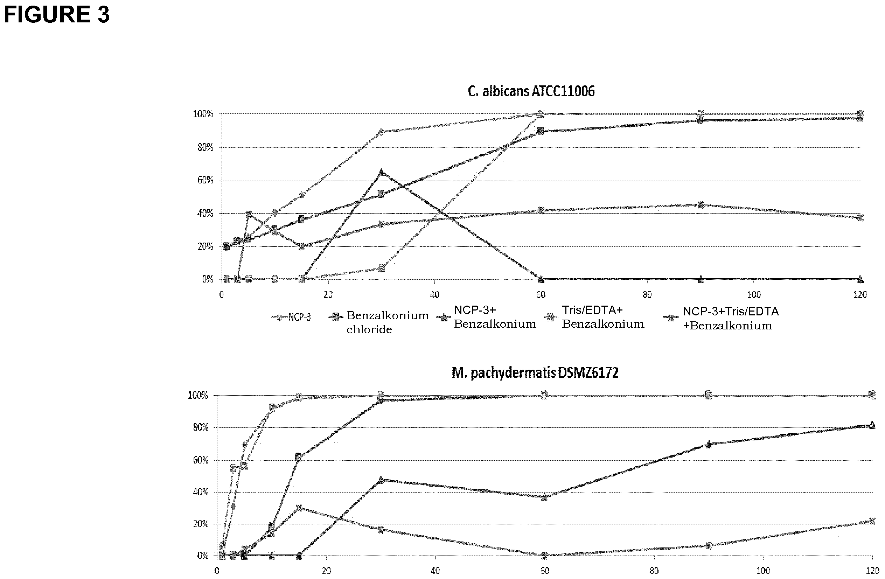

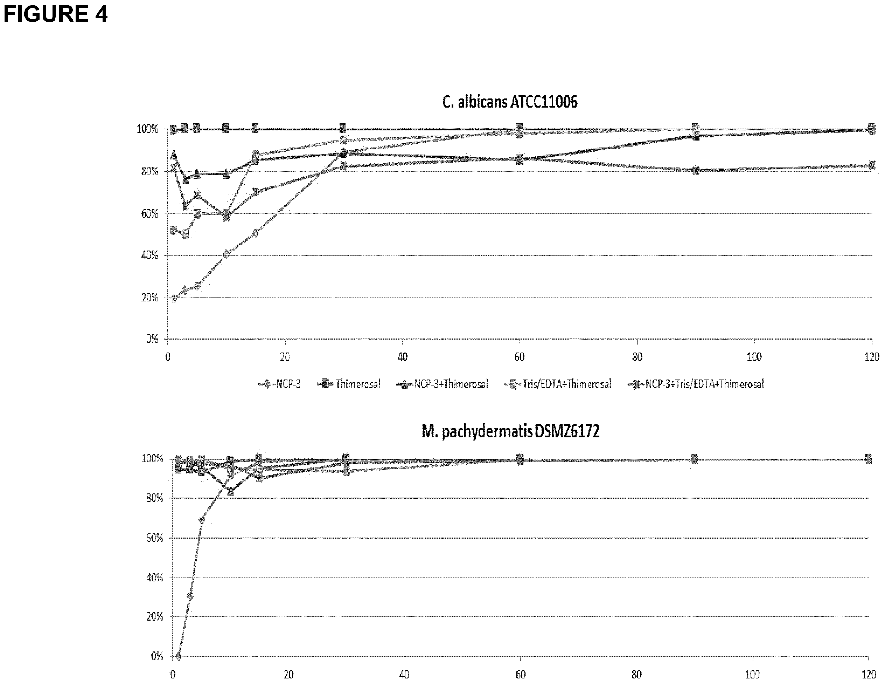

Figures 1 and 2 show all the results obtained from time killing assays towards bacterial strains *Escherichia coli* ATCC 25922, *Pseudomonas aeruginosa* ATCC 27853, *Staphylococcus aureus* ATCC 25923, Methicillin Resistant *Staphylococcus aureus* (MRSA) ATCC 43300. Figure 3 and 4 show the results obtained for fungal strains *Candida albicans* ATCC 11006 or *Malassezia pachydermatis* DSMZ 6172.

Figure 1 shows, for Gram-negative bacteria (*E. coli* and *P. aeruginosa*), a synergic increase of activity (percentage of growth inhibition) by the combination NCP-3 + Benzalkonium chloride, compared to the activity of the single components.

Conversely, for Gram-positive (MRSA and *S*. *aureus*), the three inhibition curves are very close and partly coincident: this shows that, in case of these microorganisms, the effect of the combination of the components is substantially indifferent compared to their use alone.

Figure 2 shows, in case of Gram-negative bacteria (*E. coli* and *P*. *aeruginosa*), a synergic increase, in particular in early stage of treatment, of activity (percentage of growth inhibition) by NCP-3 + Thimerosal, compared to activity of the same components used alone.

Conversely, in case of Gram-positive bacteria (MRSA and *S*. *aureus*), the three curves of inhibition are very close and partly coincident: this shows that, in case of these microorganisms, the effect of the combination of components is substantially indifferent or even antagonist compared to their use alone.

Figure 3 shows that, for fungal strains *C. albicans* and *M. pachydermatis,* the combination of peptide NCP-3 with benzalkonium chloride only, or with benzalkonium chloride + EDTA does not improve antifungal activity, but even causes a decrease of efficacy, showing an antagonistic activity in growth inhibition.

Figure 4 shows that, for fungal strains *C. albicans* and *M. pachydermatis,* the combination of the peptide NCP-3 with only Thimerosal or Thimerosal + EDTA is indifferent or worse compared to the activity of Thimerosal alone. An antagonistic or indifferent activity of these combinations of NCP-3 towards fungal growth inhibition is thus shown.

Overall, the results in Figure 1-4 show a synergic activity of the combinations of peptides and disinfectants according to the present invention, selectively against Gram-negative bacteria. The addition of the chelating agent further increases this effect.

## Claims

1. Combination of one or more antimicrobial peptides having structure (I):
KLI-X1-SKTIPA-X2-KNL-X3-YKI (I)
wherein:
- X1 is a tripeptide selected from: PIL, WIL, PIA, FIL, LIL and YIL;
- X2 is a single amino acid selected from isoleucine (I) and glycine (G);
- X3 is a single hydrophobic amino acid selected from alanine (A), phenylalanine (F), leucine (L), isoleucine (I), proline (P), tyrosine (Y), tryptophan (W) and valine (V),
with one or more disinfectants selected from benzalkonium chloride and thimerosal.

2. Combination according to claim 1, wherein X3 is selected from F, I, L and V.

3. Combination according to any one of claims 1-2, wherein said peptide is selected from:
- KLIPILSKTIPAIKNLFYKI;
- KLIWILSKTIPAIKNLFYKI;
- KLIPIASKTIPAGKNLFYKI;
- KLIPIASKTIPAGKNLIYKI;
- KLIPIASKTIPAGKNLLYKI;
- KLIPIASKTIPAGKNLVYKI;
- KLIFILSKTIPAIKNLFYKI;
- KLILILSKTIPAIKNLFYKI;
- KLIYILSKTIPAIKNLFYKI.

4. Combination according to any of claims 1-3, wherein said chelating agent comprises EDTA.

5. Pharmaceutical composition for human or veterinary use, comprising the combination described in claims 1-4, in association with pharmaceutical co-formulants.

6. Kit of parts comprising: (a) one or more antimicrobial peptides, (b) one or more disinfectants and optionally (c) one or more chelating agents, as described in the claims 1-4.

7. Combination, pharmaceutical composition or kit according to any one of claims 1-6 for use in therapy.

8. Combination, pharmaceutical composition or kit according to any one of claims 1-6 for use in a method of treating and/or preventing diseases caused by Gram-negative bacteria.

9. Combination, pharmaceutical composition or kit for use according to claim 8, wherein said Gram-negative bacteria belong to one or more of the genera: *Acinetobacter, Burkholderia, Cardiobacterium, Enterobacter, Escherichia, Flavobacterium, Haemophilus, Helicobacter, Klebsiella, Legionella, Moraxella, Mycoplasma, Neisseria, Proteus, Pseudomonas, Pseudoxanthomonas, Rickettsia, Salmonella, Shigella, Vibrio.*

## Patentansprüche

1. Kombination eines oder mehrerer antimikrobieller Peptide mit der Struktur (I):
KLI-X1-SKTIPA-X2-KNL-X3-YKI (I)
worin:
- X1 ein Tripeptid ist, das ausgewählt ist aus: PIL, WIL, PIA, FIL, LIL und YIL;
- X2 eine Einzel-Aminosäure ist, die ausgewählt ist aus Isoleucin (I) und Glycin (G);
- X3 eine hydrophobe Einzel-Aminosäure ist, die ausgewählt ist aus Alanin (A), Phenylalanin (F), Leucin (L), Isoleucin (I), Prolin (P), Tyrosin (Y), Tryptophan (W) und Valin (V),
mit einem oder mehreren desinfizierenden Mitteln, die ausgewählt sind aus Benzalkoniumchlorid und Thimerosal.

2. Kombination nach Anspruch 1, wobei X3 ausgewählt ist aus F, I, L und V.

3. Kombination nach einem der Ansprüche 1 bis 2, wobei das Peptid ausgewählt ist aus:
- KLIPILSKTIPAIKNLFYKI;
- KLIWILSKTIPAIKNLFYKI;
- KLIPIASKTIPAGKNLFYKI;
- KLlPIASKTIPAGKNLlYKI;
- KLIPIASKTIPAGKNLLYKI;
- KLlPIASKTIPAGKNLVYKI;
- KLIFILSKTIPAIKNLFYKI;
- KLILILSKTIPAIKNLFYKI;
- KLIYILSKTIPAIKNLFYKI.

4. Kombination nach einem der Ansprüche 1 bis 3, wobei der Chelatbildner EDTA aufweist.

5. Pharmazeutische Zusammensetzung zur humanmedizinischen oder veterinärmedizinischen Verwendung, aufweisend die in den Ansprüchen 1 bis 4 beschriebene Kombination in Verbindung mit pharmazeutischen Co-Formulierungsmitteln.

6. Kit-of-Parts aufweisend: (a) ein oder mehrere antimikrobielle Peptide (b) ein oder mehrere desinfizierende Mittel und optional (c) ein oder mehrere Chelatbildner, wie in den Ansprüchen 1 bis 4 beschrieben.

7. Kombination, pharmazeutische Zusammensetzung oder Kit nach einem der Ansprüche 1 bis 6 zur therapeutischen Verwendung.

8. Kombination, pharmazeutische Zusammensetzung oder Kit nach einem der Ansprüche 1 bis 6 zur Verwendung in einem Verfahren zur Behandlung und/oder Verhütung von Erkrankungen, die durch gramnegative Bakterien verursacht werden.

9. Kombination, pharmazeutische Zusammensetzung oder Kit zur Verwendung nach Anspruch 8, wobei gramnegativen Bakterien zu einer oder mehreren der Gattungen *Acinetobacter, Burkholderia, Cardiobacterium, Enterobacter, Escherichia, Flavobacterium, Haemophilus, Helicobacter, Klebsiella, Legionella, Moraxella, Mycoplasma, Neisseria, Proteus, Pseudomonas, Pseudoxanthomonas, Rickettsia, Salmonella, Shigella, Vibrio* gehören.

## Revendications

1. Combinaison d'un ou plusieurs peptides antimicrobiens de structure (I):
KLI-X1-SKTIPA-X2-KNL-X3-YKI (I)
dans laquelle:
- X1 est un tripeptide choisi parmi : PIL, WIL, PIA, FIL, LIL et YIL
- X2 est un unique acide aminé choisi parmi l'isoleucine (I) et la Glycine (G);
- X3 est un unique acide aminé hydrophobe choisi parmi l'alanine (A), la phénylalanine (F), la leucine (L), l'isoleucine (I), la proline (P), la tyrosine (Y), le tryptophane (W) et la valine (V),
avec un ou plusieurs désinfectants choisis parmi le chlorure de méthylbenzéthonium et le timérosal.

2. Combinaison selon la revendication **1,** dans laquelle X3 est choisi parmi : F, I, L et V.

3. Combinaison selon l'une quelconque des revendications **1-2,** dans laquelle ledit peptide est choisi parmi :
- KLIPILSKTIPAIKNLFYKI;
- KLIWILSKTIPAIKNLFYKI;
- KLIPIASKTIPAGKNLFYKI;
- KLIPIASKTIPAGKNLIYKI;
- KLIPIASKTIPAGKNLLYKI;
- KLIPIASKTIPAGKNLVYKI;
- KLIFILSKTIPAIKNLFYKI;
- KLILILSKTIPAIKNLFYKI;
- KLIYILSKTIPAIKNLFYKI.

4. Combinaison selon l'une quelconque des revendications **1-3,** dans laquelle ledit agent chélatant comprend de l'EDTA.

5. Composition pharmaceutique à usage humain ou vétérinaire, comprenant la combinaison décrite dans les revendications **1-4,** en association avec des coformulants pharmaceutiques.

6. Kit de pièces comprenant : (a) un ou plusieurs peptides antimicrobiens, (b) un ou plusieurs désinfectants et éventuellement (c) un ou plusieurs agents chélateurs, comme décrit dans les revendications **1-4.**

7. Combinaison, composition pharmaceutique ou kit selon l'une quelconque des revendications **1-6** pour une utilisation en thérapie.

8. Combinaison, composition pharmaceutique ou kit selon l'une quelconque des revendications **1-6** à utiliser dans un procédé de traitement et / ou de prévention de maladies causées par des bactéries à Gram négatif.

9. Combinaison, composition pharmaceutique ou kit à utiliser selon la revendication **8,** dans laquelle lesdites bactéries à Gram négatif appartiennent à un ou plusieurs des genres : *Acinetobacter, Burkholderia, Cardiobacterium, Enterobacter, Escherichia, Flavobacterium, Haemophilus, Helicobacter, Klebsiella, Legionella, Moraxella, Mycoplasme, Neisseria, Proteus, Pseudomonas, Pseudoxanthomonas, Rickettsia, Salmonella, Shigella, Vibrio.*
